# EUROPEAN PATENT APPLICATION

(11) **EP 2 623 145 A1**
(43) Date of publication of application: **07.08.2013**
(21) Application number: 12000736.4
(22) Date of filing: 03.02.2012
(51) Int. Cl.: A61M 5/32

(54) **Retractable needle apparatus**

(71) Applicant: Q Stat Pty Ltd, Hawthorn East, VIC 3123 (AU)
(72) Inventor: Davenport, Thomas, Hawthorn East, Victoria 3123 (AU)
(74) Representative: Polo Flores, Luis Miguel

(57) **Abstract**

A syringe cover (10) for providing needle retraction capabilities for a prefilled syringe, the prefilled syringe (1) including an extended needle (5) at a first distal end interconnecting a fluid cavity (6) containing a fluid to be expelled, the cover including: a chamber within the cover for receipt of the prefilled syringe; a first catch (15) for engaging a syringe lip (4) on the prefilled syringe, holding the syringe in a needle extended position; a translation (11) means for translating the needle from a needle extended position to a needle retracted position wherein the needle is substantially within the chamber; a plunger (18) for expelling fluids from a cavity within the syringe by translation towards the distal end of the syringe, the plunger including a first (20) catch release mechanism thereon; wherein upon movement of the plunger towards the distal end of the syringe, the first catch release mechanism (20) engages the first catch (15,16) to release the syringe from a needle extended position and the translation means translates the syringe to a needle retracted position.

## Description

### FIELD OF THE INVENTION

The present invention relates to the field of retractable syringes and in particular, discloses a unique and modified form for dealing with single dose syringes.

### BACKGROUND OF THE INVENTION

Many attempts have been made to provide for retractable syringe type technology when dealing with pre-filled syringes of the type traditionally provided in a glass ampoule or the like.

One early patent is United States Patent No. 4624660 to Mijers et al which deals with a retractable needle arrangement for a glass ampoule. Other examples include United States Patent No. 5624400 to Firth et al., United States Patent No. 5634909 to Schmitz. These applications are amongst many that provide for ampoule based retractable needle operation where a standard ampoule is provided and retractable needle operations are carried out around the standard ampoule.

The problem with prior art devices is that they can be cumbersome and expensive to manufacture requiring the interaction of multiple parts.

Any discussion of the prior art throughout the specification should in no way be considered as an admission that such prior art is widely known or forms part of common general knowledge in the field.

### SUMMARY OF THE INVENTION

It is an object of the present invention to provide an improved form of retractable syringe arrangement for use with pre-filled syringes.

In accordance with a first aspect of the present invention, there is provided a syringe cover for providing needle retraction capabilities for a prefilled syringe, the prefilled syringe including an extended needle at a first distal end interconnecting a fluid cavity containing a fluid to be expelled, the cover including: a chamber within the cover for receipt of the prefilled syringe; a first catch for engaging a syringe lip on the prefilled syringe, holding the syringe in a needle extended position; a translation means for translating the needle from a needle extended position to a needle retracted position wherein the needle is substantially within the chamber; a plunger for expelling fluids from a cavity within the syringe by translation towards the distal end of the syringe, the plunger including a first catch release mechanism thereon; wherein upon movement of the plunger towards the distal end of the syringe, the first catch release mechanism engages the first catch to release the syringe from a needle extended position and the translation means translates the syringe to a needle retracted position.

The cover further preferably can include a second catch for holding the syringe substantially permanently in a needle retracted position after translation by the translation means. The second catch preferably can include a first tab for engaging the syringe lip in the needle retract position hindering continued translation by the translation means. The second catch preferably can include a second tab for engaging the syringe lip in the needle retracted position so as to hinder movement towards the distal end of the cover.

### BRIEF DESCRIPTION OF THE DRAWINGS

Preferred forms of the present in invention will now be described with reference to the accompanying drawings in which:

Fig. 1 illustrates a side perspective view of a pre-filled syringe without a plunger attached;

Fig. 2 illustrates a sectional view of a pre-filled syringe without a plunger attached;

Fig. 3 illustrates a side perspective view of the cover portions of the preferred embodiment;

Fig. 4 illustrates a sectional view of the cover portion of the preferred embodiment;

Fig. 5 illustrates the cover in pre-filled form;

Fig. 6 illustrates a section view of the cover in pre-filled form;

Fig. 7 illustrates a spring;

Fig. 8 illustrates a plunger;

Fig. 9 and Fig. 10 illustrate sectional views of an assembled form of the preferred embodiment;

Fig. 11 illustrates a side perspective view of the preferred embodiment just before utilisation;

Fig. 12 illustrates a sectional view of the preferred embodiment just before - utilisation;

Fig. 13 is a side perspective view of the preferred embodiment showing utilisation;

Fig. 14 is a sectional view of the preferred embodiment near the end of utilisation;

Fig. 15 illustrates a side perspective view at the end of utilisation;

Fig. 16 illustrates a sectional view at the end of utilisation;

Fig. 17 illustrates the retraction mechanism of the preferred embodiment in a nearly retracted and locked state as does Fig. 18; and

Fig. 19 and Fig. 20 illustrate sectional views at the end of the retraction activation process illustrating the locking of the pre-filled syringe device in a fully retracted state.

### DESCRIPTION OF PREFERRED AND OTHER EMBODIMENTS

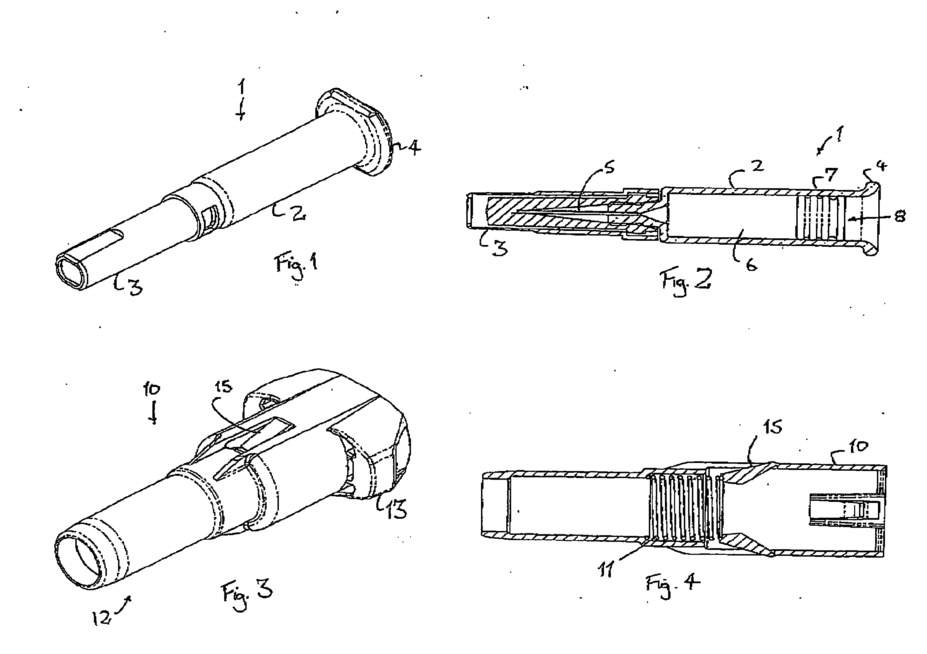

The preferred embodiment provides for retractable syringe operation for a pre- filled disposable syringe. Pre-filled disposable syringes are becoming increasingly common where expensive drugs are utilised as they minimise the drug left in the syringe after use. Also the drug is normally provided in a pre-filled syringe in a precise dose minimising dosing errors and the time required to prepare an injection. For example, Fig. 1 illustrates a common form ofpre-filled syringe which includes a body 2 and a tip 3 which includes a cap and a needle. The body 2 normally includes some form of lip 4 at a distal end to the cap 3 or needle.

Fig. 2 illustrates a sectional view along the syringe 1 of Fig. 1. The pre-filled syringe includes a needle 5 in fluid communication with a reservoir 6 which contains the drug to be utilised. The reservoir 6 includes a stopper 7 at one end thereof which is moveable along the reservoir. The stopper 7 is normally profiled along an internal surface 8 so as to receive a mating plunger. Normally, the mating plunger is screwed into the cavity 8 during the last stage of manufacture. The single use pre-filled syringe is utilised after the cap 3 has been removed. Of course, it is well known that the arrangement 1 lacks the advantages of a retractable syringe and it is to this area that the preferred embodiment is addressed. The preferred embodiment provides single use retractable syringe capabilities for the pre-filled syringe 1 of Fig. 1 minimizing the risk of a needlestick injury to the user.

Turning now to Fig. 3, there is illustrated the one piece cover 10 of the preferred embodiment in a prepackaged form before the insertion of the pre-filled syringe 1. Fig. 4 illustrates a sectional view through the arrangement of Fig. 3 wherein it is noted the cover 10 is formed from one piece by injection moulding and has been fitted with a compressed internal spring 11. Turning to Fig. 3, the cover 10 generally includes a cylindrical portion 12 and finger grip 13.

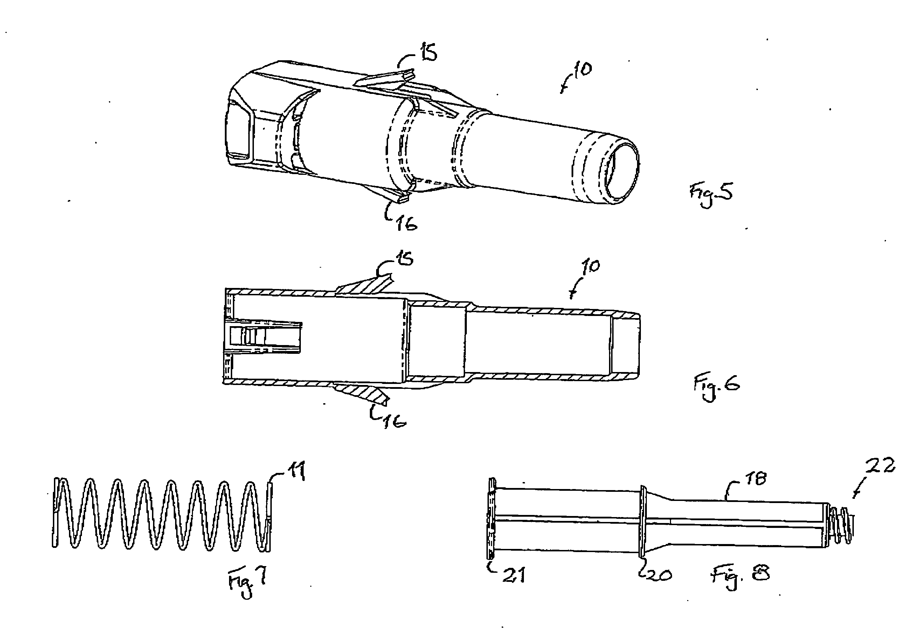

In Fig. 5 and Fig. 6, there are illustrated views of the cover 10 before the insertion of internal spring 11. This spring 11, illustrated in Fig. 7 is inserted and the tabs 15,16 activated by bending inwards so as to catch the spring 11.

In Fig. 8 there is illustrated a side plan view of the plunger 18 which includes two annular rings 20, 21 and a screw end 22 for mating with stopper cavity 8 of the prefilled syringe 1.

Fig. 5 and Fig. 6 illustrate the cover as manufactured. Fig. 7 illustrates the spring 11 before utilisation. Fig. 8 illustrates the plunger 18 which includes the annular rings 20, 21 and screw end 22.

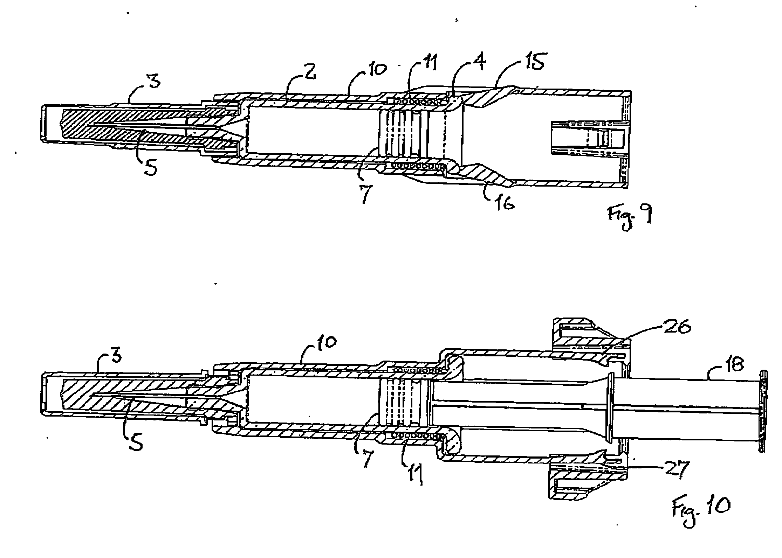

The pre-filled syringe is inserted in the cover 10 and the tabs 15,16 are utilised to engage the rim 4 of the pre-filled syringe as illustrated in Fig. 9. The spring 11 is held under compression. The tabs 15, 16 are depressed so as to engage the rim 4 and the plunger 18 is inserted into the internal surface cavity of the plug 8 and screwed into the cavity. The arrangement of Fig. 10 can be packaged for dispatch to a site where it is to be used. The sectional axis of Fig. 9 and Fig. 10 are substantially orthogonal.

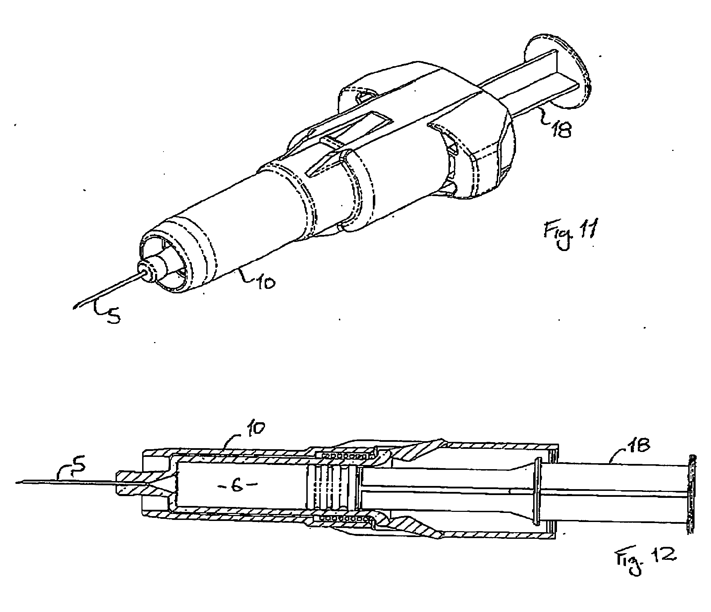

When it comes time to utilise the syringe, the cap is removed as illustrated in Fig. 11 and Fig. 12. The needle 5 is inserted into the patient and the plunger 18 depressed so that the fluid within the chamber 6 is dispensed in the usual manner.

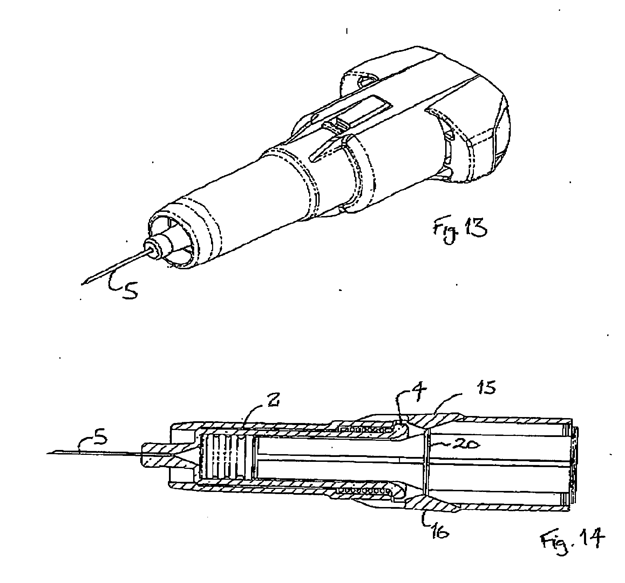

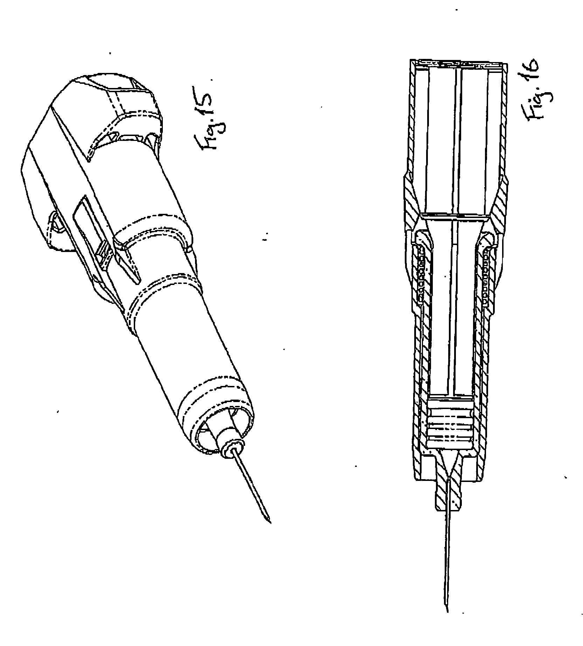

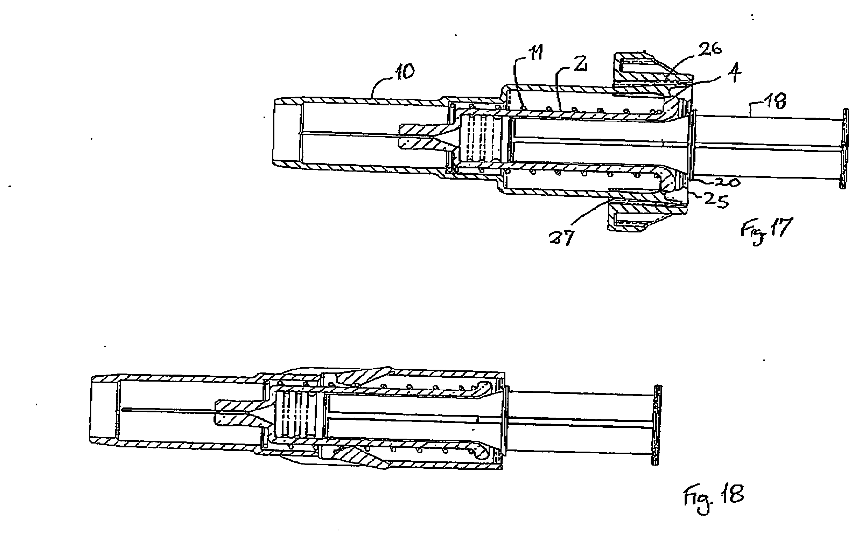

Figs. 13 and Fig. 14 illustrate the position as the plunger has been inserted a substantial part of its way into the cavity 6. Near the end of the plunger's stroke, the annular ring 20 engages tabs 15-16, as illustrated in Fig. 14, pushing them out past the lip 4 of the body 2 thereby "activating" the needle retraction process. Figs. 15 and Fig. 16 illustrate the plunger substantially at the end of its stroke. Upon release of tabs 15-16, the body 2 can move under the influence of spring 11. The body 2 and attached needle 5 then retract within the cover 10. The body 2 continues to move until rim 4 of body 2 engages rim 25 of the cover 10 as illustrated in Fig. 17. Fig. 18 illustrates a sectional view of another axis of the preferred embodiment.

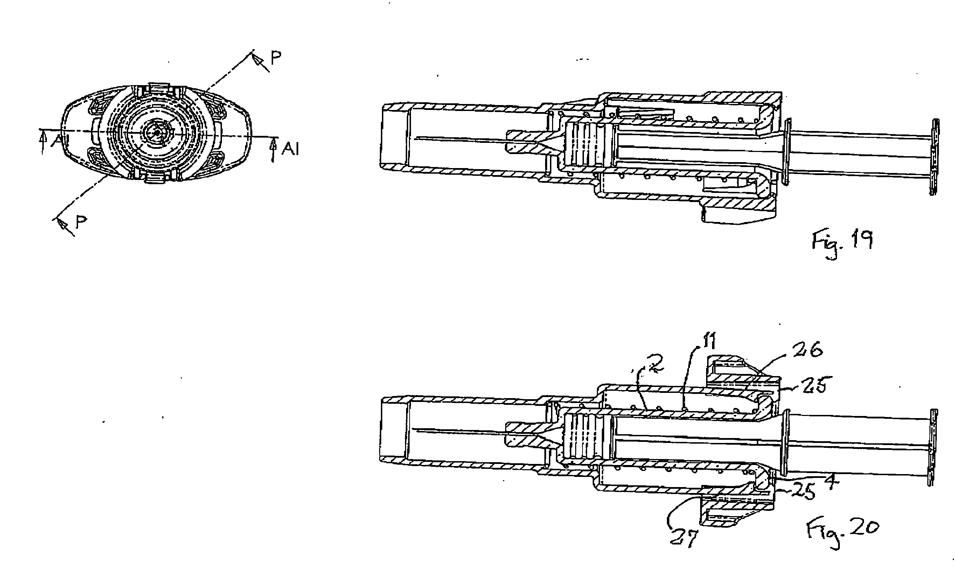

As illustrated in Fig. 17, as the body 2 moves distally, the rim 4 begins to be engaged by a second set of tabs 26, 27. These tabs are normally sprung inward and they are resiliently deflected by the rim 4. Upon the rim 4 passing, they move to engage the rim as illustrated in Fig. 20 wherein the body 2 is firmly held fixed between the rim 25 and the tabs 26, 27 by engagement of the lip 4. Hence, the body 2 is firmly held in a retracted position. The final retracted as illustrated in Fig. 19 and Fig. 20 with the body 2 firmly held in a retracted position. This aids and assists in safe disposable of the syringe after utilisation and minimises the risk of a needlestick injury to the user.

Although the invention has been described with reference to specific examples it will be appreciated by those skilled in the art that the invention may be embodied in many other forms.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

The reference to any prior art in this specification is not and should not be taken as an acknowledgement or any form of suggestion that the prior art forms part of the common general knowledge.
The claims defining the invention are as follows:

## Claims

1. A syringe cover for providing needle retraction capabilities for a pre-filled syringe, the pre-filled syringe including an extended needle at a first distal end interconnecting a fluid cavity containing a fluid to be expelled, the cover including:
a chamber within the cover for receipt of the pre-filled syringe;
a first catch for engaging a syringe lip on the pre-filled syringe, holding the syringe in a needle extended position;
a translation means for translating the needle from a needle extended position to a needle retracted position wherein the needle is substantially within said chamber;
a plunger for expelling fluids from a cavity within the syringe by translation towards the distal end of the syringe, the plunger including a first catch release mechanism thereon;
wherein upon movement of said plunger towards the distal end of the syringe, said first catch release mechanism engages said first catch to release the syringe from a needle extended position and said translation means translates the syringe to a needle retracted position,

2. A cover as claimed in claim 1 wherein:
said cover further includes a second catch for holding the syringe substantially permanently in a needle retracted position after translation by the translation means.

3. A cover as claimed in claim 2 wherein said second catch includes a first tab for engaging the syringe lip in the needle retract position hindering continued translation by the translation means.

4. A cover as claimed in claim 2 wherein said second catch includes a second tab for engaging the syringe lip in the needle retracted position so as to hinder movement towards the distal end of the cover.

5. A syringe cover substantially as herein described with reference to any one of the embodiments of the invention illustrated in the accompanying drawings and/or examples.

6. A pre-filled retractable syringe substantially as herein described with reference to any one of the embodiments of the invention illustrated in the accompanying drawings and/or examples
